# EUROPEAN PATENT APPLICATION

(11) **EP 2 293 654 A2**
(43) Date of publication of application: **09.03.2011**
(21) Application number: 10174699.8
(22) Date of filing: 31.08.2010
(51) Int. Cl.: H05G 1/28

(54) **Radiography management system and method**

(30) Priority: 01.09.2009 JP 2009201277
(71) Applicant: Fujifilm Corporation, Minato-ku Tokyo (JP)
(72) Inventor: Ohta, Yasunori, Kanagawa (JP); Heki, Tatsuo, Kanagawa (JP); Kuwabara, Takao, Kanagawa (JP); Iwasaki, Nobuyuki, Kanagawa (JP); Kusunoki, Tetsurou, Kanagawa (JP); Satoh, Kimihiko, Kanagawa (JP); Nishino, Naoyuki, Kanagawa (JP)
(74) Representative: Klunker . Schmitt-Nilson . Hirsch

(57) **Abstract**

When radiography is performed on a patient (S) at a radiography apparatus (10), first, a past collected radiation dose for the patient (S) and a past collected radiation dose for a region of the patient (S) on which radiography will be performed are extracted from a database. Judgment is made as to whether the collected radiation doses are greater than or equal to set threshold values. If it is judged that the collected radiation doses are greater than or equal to the set threshold values, the radiography apparatus (10) outputs an alert for a radiographer. If it is judged that the collected radiation doses are less than or equal to the set threshold values, radiography is started by the radiography apparatus (10).

## Description

### BACKGROUND OF THE INVENTION

### Field of the Invention

The present invention relates to a radiography management system and method for managing a radiation dose for a patient when radiography is performed on the patient.

### Description of the Related Art

Conventionally, diagnosis was performed by using radiographic images of the inside of a human body obtained by chest X-ray radiography or by radiography using a mammography apparatus, a CT (computed tomography) apparatus or the like. In radiography, the human body is irradiated with radiation. Therefore, although the dose of radiation in radiography is small, it is necessary to manage the dose of radiation for the safety of the human body (patient). As the method for managing radiography, various methods have been proposed (please refer to Japanese Unexamined Patent Publication No. 2008-284294 (Patent Document 1) and Japanese Unexamined Patent Publication No. 2006-218139 (Patent Document 2), for example).

Patent Document 1 considers the fact that radiography is performed at various medical institutions (hospitals), and proposes a technique of managing the dose of radiation in total by collecting, through a local medical network, the dose of radiation performed at each hospital and the like. Further, Patent Document 2 discloses storage of a log at the time of failed radiography in such a manner to relate the log to a radiographic image. The log at the time of failed radiography can be referred to any time.

However, in Patent Document 1, the dose of radiation is managed based on the radiation dose that is stored as supplementary information for a radiographic image that is used for diagnosis (image diagnosis). Therefore, it is impossible to accurately manage the dose of radiation. Specifically, when radiography is performed, a patient is irradiated with radiation not only in main radiography (actual radiography in which an image for diagnosis is obtained) for obtaining an image for diagnosis but also in pre-radiation, failed radiography, and the like. Pre-radiation is performed to check the location of a diseased part (lesion) or the like before main radiography is performed. In failed radiography, radiography fails, and a desirable radiographic image is not obtained. However, when only the radiation dose at the time of obtainment of a radiographic image for diagnosis is managed as in Patent Document 1, it is impossible to manage the radiation dose in pre-radiation or the like, which is not recorded. Therefore, it is impossible to accurately manage the dose of radiation irradiated a patient.

### SUMMARY OF THE INVENTION

In view of the foregoing circumstances, it is an object of the present invention to provide a radiography management system and method that can accurately detect and manage the dose of radiation irradiated a patient.

A radiography management system of the present invention is a radiography management system for managing a radiation dose in radiography performed at each of a plurality of radiography apparatuses that are connected to a network in such a manner that data is transmittable through the network, the system comprising:
a radiation dose detection means that detects the dose of radiation in radiography that has irradiated a patient from the start to the end of radiography to obtain a radiographic image at each of the plurality of radiography apparatuses; and
a radiation dose management means that obtains, through the network, the dose of radiation detected by the radiation dose detection means, and that stores and manages, for each patient, a plurality of doses of radiation obtained from the plurality of radiography apparatuses respectively in each time of radiography.

A radiography management method of the present invention is a radiography management method for managing a radiation dose in radiography performed at each of a plurality of radiography apparatuses that are connected to a network in such a manner that data is transmittable through the network, the method comprising the steps of:
detecting the dose of radiation in radiography that has irradiated a patient from the start to the end of radiography to obtain a radiographic image at each of the plurality of radiography apparatuses;
obtaining, through the network, the detected dose of radiation in radiography; and
storing and managing, for each patient, a plurality of doses of radiation obtained from the plurality of radiography apparatuses respectively in each time of radiography.

Here, the radiography apparatus is not particularly limited as long as the apparatus obtains (detects) a radiographic image by detecting radiation that has passed through a subject (patient). For example, the radiography apparatus may be a chest X-ray radiography apparatus, a mammography apparatus, a radiography apparatus for dentistry, a CT apparatus for observing internal organs of patients, and the like.

Further, the term "dose of radiation in radiography" means the total dose of radiation obtained by adding the doses of radiation in radiography from the start to the end of radiography. Therefore, the dose of radiation in radiography is not limited to the dose of radiation during detection of a radiographic image. The dose of radiation in radiography refers to the total dose of radiation including the dose of radiation in pre-radiation before main radiography if pre-radiation has been performed, the dose of radiation in failed radiography if radiography has failed, and the dose of radiation in main radiography in which the radiographic image is detected.

The radiation dose management means may include a data analysis means that judges whether a collected radiation dose obtained by adding the plurality of doses of radiation for each patient is greater than or equal to a predetermined set threshold value. Further, each of the plurality of radiography apparatuses may have an alert output means that outputs an alert in the case that the data analysis means has judged that the collected radiation dose is greater than or equal to the predetermined set threshold value.

The radiation dose management means may have a function for managing the collected radiation dose for each radiation-irradiated region of each patient in addition to a function for calculating a collected radiation dose by adding the doses of radiation at a plurality of times of radiography for each patient.

According to the radiography management system and method of the present invention, a plurality of radiography apparatuses are connected to a network in such a manner that data is transmittable through the network, and a radiation dose in radiography at each of the plurality of radiography apparatuses is managed. When a radiographic image is obtained by irradiating a patient with radiation at each of the plurality of radiography apparatuses, the dose of radiation in radiography that has irradiated the patient from the start to the end of radiography is detected. Further, the detected dose of radiation in radiography is obtained through the network. Further, the detected dose of radiation in radiography is stored and managed, for each patient, in each time of radiography. Therefore, it is possible to detect and manage the dose of radiation irradiated the patient in pre-radiation, failed radiography or the like, in which a radiographic image for diagnosis is not obtained, in addition to the dose of radiation stored in such a manner to be related to a radiographic image, which is obtained in main radiography. Therefore, it is possible to manage the radiation for a patient based on an accurate radiation dose for the patient.

When the radiation dose management means includes a data analysis means that judges whether a collected radiation dose obtained by adding the plurality of doses of radiation for each patient is greater than or equal to a predetermined set threshold value, and each of the plurality of radiography apparatuses has an alert output means that outputs an alert in the case that the data analysis means has judged that the collected radiation dose is greater than or equal to the predetermined set threshold value, it is possible to output an alert in the case that the radiation dose for the patient has increased or exceeded a certain level, thereby making a radiographer or radiologist notice the increase of the radiation dose.

When the radiation dose management means has a function for managing the collected radiation dose for each radiation-irradiated region of each patient, it is possible to manage the radiation dose for each region of the human body.

Further, when the radiation dose detection means detects the dose of radiation in radiography by adding the dose of radiation in pre-radiation if pre-radiation has been performed, the dose of radiation in failed radiography if radiography has failed, and the dose of radiation in main radiography in which a radiographic image is obtained, it is possible to detect and manage, as a radiation dose for a patient, the radiation dose for the patient in pre-radiation and failed radiography, in which a radiographic image for diagnosis is not obtained, in addition to the radiation dose that is stored in such a manner to be related to the radiographic image.

### BRIEF DESCRIPTION OF THE DRAWINGS

Figure 1 is a schematic diagram illustrating a radiography management system according to an embodiment of the present invention;
Figure 2 is a schematic diagram illustrating an example of a radiography apparatus connected to the radiography management system illustrated in Figure 1;
Figure 3A is a schematic diagram illustrating an example of radiation timing detected by a radiation dose detection means illustrated in Figure 2;
Figure 3B is a schematic diagram illustrating another example of radiation timing detected by a radiation dose detection means illustrated in Figure 2;
Figure 4 is a schematic diagram illustrating an example of data structure in a database illustrated in Figure 1; and
Figure 5 is a flow chart illustrating a radiography management method according to an embodiment of the present invention.

### DESCRIPTION OF THE PREFERRED EMBODIMENTS

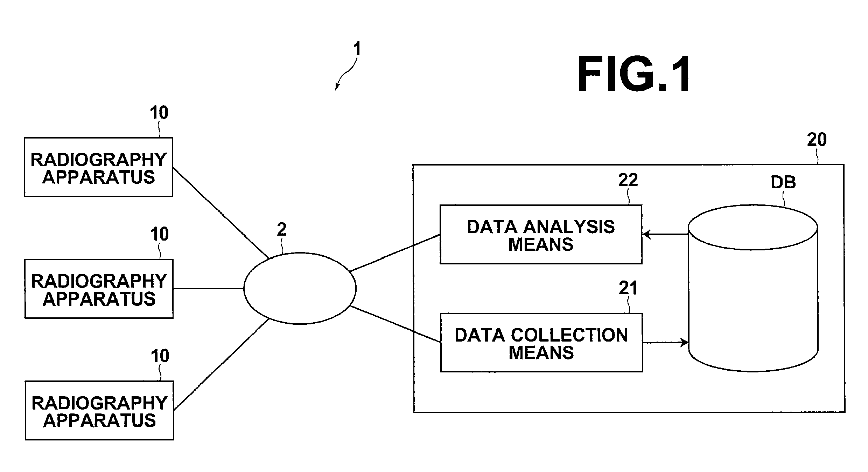

Hereinafter, embodiments of the present invention will be described in detail with reference to drawings. Figure 1 is a schematic diagram illustrating a radiography management system according to an embodiment of the present invention. The radiography management system 1 manages a radiation dose for each patient by integrating (collecting and combining) doses of radiation by radiography apparatuses 10 installed at various hospitals. In the radiography management system 1, the plurality of radiography apparatuses 10 and a radiation dose management means 20 are connected through a network 2 in such a manner that data can be transmitted therebetween.

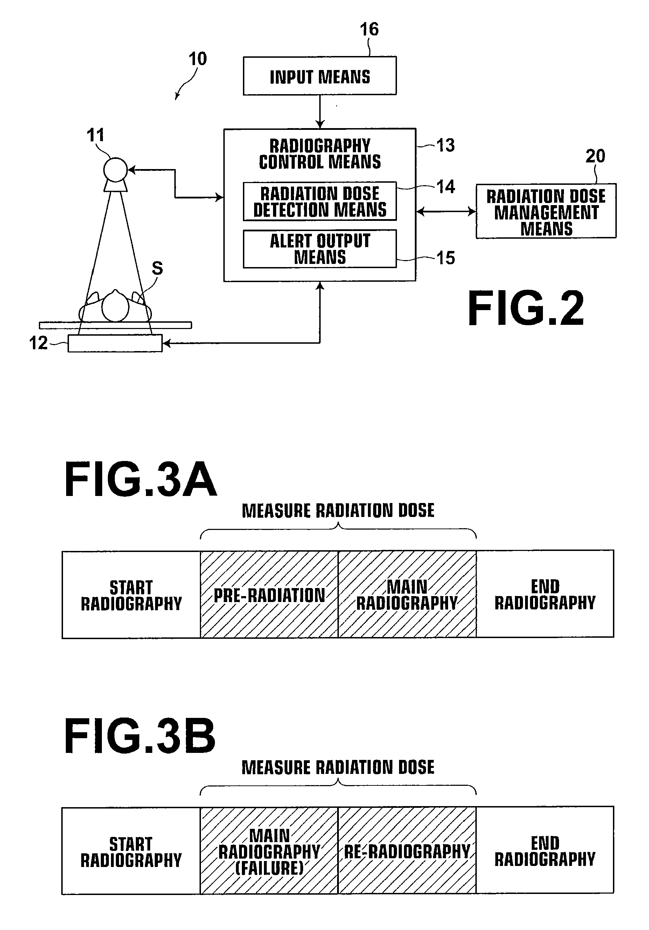

Figure 2 is a schematic diagram illustrating an example of the radiography apparatus 10. The radiography apparatus 10 illustrated in Figure 2 is a decubitus-type radiography apparatus, and includes a radiation source 11 and a radiation detector 12. In the radiography apparatus 10, radiation is output from the radiation source 11 to subj ect S. Further, the radiation that has passed through the subject S is detected, as a radiographic image, by the radiation detector 12. The radiation source 11 and the radiation detector 12 are controlled by a radiography control means 13.

The radiography control means 13 includes a radiation dose detection means 14 and an alert output means 15. The radiation dose detection means 14 detects the dose of radiation irradiated subject S from the start of preparation of radiography till the end of radiography when a radiographic image of the subject S is obtained by irradiating the subject S with radiation output from the radiography apparatus 10. Here, the radiation dose detection means 14 may calculate the radiation dose based on the control condition (the kind of a radiation source, a tube voltage, a tube current, or the like) of the radiography control means 13. Alternatively, the radiation dose detection means 14 may calculate the radiation dose based on the density of a portion of the radiographic image, the portion in which the subject is not present. It is not necessary that the radiation dose detection means 14 calculates the radiation dose by signal processing. The radiation dose detection means 14 may be a radiation counter provided at a radiation output portion of the radiation source 11 or at an outer edge or end of the radiation detector 12, and the radiation may be directly measured by the radiation counter.

For example, as illustrated in Figure 3A, there are cases in which a pre-radiation image is obtained by performing pre-radiation before main radiography, and after the condition of radiography is checked by using the pre-radiation image, main radiography is performed to obtain a radiographic image. In such cases, the radiation dose detection means 14 adds the radiation dose in the main radiography and the radiation dose in pre-radiation to detect the dose of radiation in radiography.

Further, for example, as illustrated in Figure 3B, there are cases in which a desirable radiographic image is not detected in the first radiography operation (a failure, or failed radiography) and radiography is performed again (re-radiography) to obtain a desirable radiographic image. In such cases, the radiation dose detection means 14 adds the radiation dose in re-radiography and the radiation dose in the failed radiography to detect the dose of radiation in radiography. Further, when a main radiography operation that has been performed after pre-radiation is a failure, and a second (or more) radiography operation has been performed, the dose of radiation in the pre-radiation, the dose of radiation in the failed radiography operation, and the dose of radiation in the second (or more) radiography operation are added to detect the dose of radiation in radiography. Specifically, the radiation dose detection means 14 detects the radiation dose from the time when the radiography apparatus 10 has become ready for radiography of a patient till the time when the patient leaves the radiography apparatus 10.

The alert output means 15 illustrated in Figure 2 outputs an alert to make a radiographer pay attention to the dose of radiation for the patient. For example, the alert output means 15 outputs an alert, for example, by using a display unit or screen, or by voice, sound, or the like.

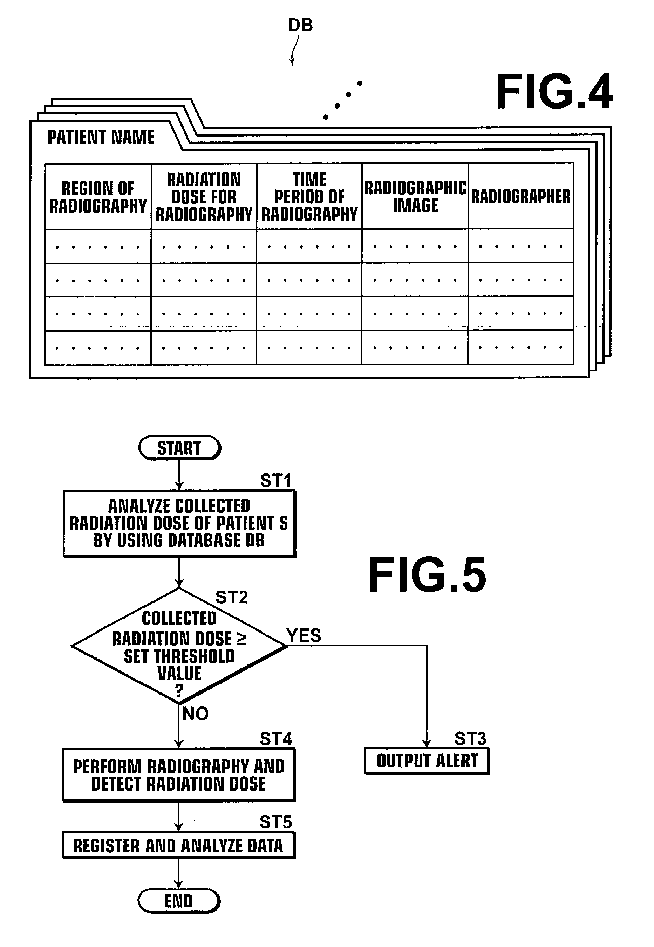

The radiation dose management means 20 illustrated in Figure 1 adds radiation doses detected by the radiation dose detection means 14 for each patient, and stores and manages the radiation dose. The radiation dose management means 20 includes a data collection means 21, a data analysis means 22, and a database DB. The data collection means 21 has a function for adding radiation doses in radiography transmitted from the plurality of radiography apparatuses 10 through the network 2 to obtain a collected radiation dose for each patient S, and a function for registering the collected radiation dose in the database DB. Figure 4 is a schematic diagram illustrating an example of the data structure of the database DB. The database DB stores a data table for each patient. The data table for each patient stores the time period of radiography, a region (site) of the patient irradiated with radiation, a radiation dose of radiography, radiographic image data, radiographer information, and the like, which are registered by the data collection means 21.

The data analysis means 22 illustrated in Figure 1 analyzes various kinds of data registered in the database DB. Specifically, the data analysis means 22 has a function for calculating, as a collected radiation dose, the total amount of radiation doses for each patient and a function for judging whether the collected radiation dose is greater than or equal to a predetermined set threshold value. When the collected radiation dose is greater than or equal to the predetermined set threshold value, the information that the collected radiation dose is greater than or equal to the predetermined set threshold value is sent to the radiography apparatus 10 through the network 2. When the radiography apparatus 10 receives the information, the alert output means 15 of the radiography apparatus 10 outputs an alert for the radiographer.

Further, the data analysis means 22 may have a function for analyzing data in the database DB for each radiographer who has performed radiography. For example, the data analysis means 22 may have a function for extracting the frequency of occurrence of failed radiography for each radiographer. When the frequency of occurrence of failed radiography exceeds a predetermined value, the information that the frequency of occurrence of failed radiography has exceeded the predetermined value may be reported to a chief radiographer or the like by the alert output means 15. Accordingly, it is possible to monitor the radiation dose for each patient so that the radiation dose does not increase excessively. Further, the data collection means 21 may have a function for registering, in the database DB, a reason for failed radiography that is input through an input means 16 when radiography has failed. Further, the data analysis means 22 may evaluate the operation of each radiographer, excluding factors, such as the body movement of a patient, which are irrelevant to the ability or performance of the radiographers by referring to the reason for failed radiography. Further, the data analysis means 22 may calculate the evaluation value of a radiographer, taking the time period of radiography into consideration in addition to the occurrence of failed radiography.

Since the total radiation dose from the start of radiography till the end of radiography is detected, as a radiation dose for radiography, and managed, it is possible to manage the radiation dose for each patient based on an accurate radiation dose. Conventionally, the radiation dose was managed based on the radiation dose related to a radiographic image. When the radiation dose is managed in such a manner, the radiation dose in pre-radiation, the radiation dose in failed radiography, and the like are unknown. Therefore, it is impossible to accurately mange the radiation dose for patient S. In contrast, when the radiation dose detection means 14 detects the total radiation dose from the start of radiography till the end of radiography, it is possible to accurately manage the dose of radiation with which the subject S has been irradiated.

Figure 5 is a flow chart illustrating a radiation dose management method according to an embodiment of the present invention. The radiation dose management method will be described with reference to Figures 1 through 5. First, information about patient S, on which radiography is going to be performed, is input. The information about the patient S and radiography region information are sent to the radiation dose management means 20 through the network 2. The data analysis means 22 in the radiation dose management means 20 extracts past collected radiation dose for the patient S and past collected radiation dose for the region of the patient S to be radiographed from the database DB (step ST1).

After then, judgment is made as to whether the collected radiation doses are greater than or equal to respective set threshold values (step ST2) . When it is judged that at least one of the collected radiation dose is greater than or equal to the set threshold value, the data analysis means 22 sends alert information to the radiography apparatus 10. Further, the alert output means 15 in the radiography apparatus 10 outputs an alert for the radiographer (step ST3).

When it is judged that the collected radiation doses are less than the set threshold value, radiography by the radiography apparatus 10 is started (step ST4) . At this time, the radiation dose detection means 14 adds not only the radiation dose in main radiography but the radiation dose in pre-radiation and/or the radiation dose in failed radiography, which is failed main radiography. When radiography ends, the radiation dose of radiography detected by the radiation dose detection means 14 is sent to the radiation dose management means 20 through the network 2 (step ST5) . After then, the data collection means 21 of the radiation dose management means 20 updates a data sheet of the patient S by using data obtained in the present radiography.

According to the above embodiments, the plurality of radiography apparatuses 10 are connected through a network in such a manner that data can be transmitted therefrom. Further, a radiation dose in radiography by each of the plurality of radiography apparatuses 10 is managed. When a patient is irradiated with radiation in each of the radiography apparatuses 10 to detect a radiographic image of the patient, the dose of radiation for radiography, i.e., the radiation with which the patient has been irradiated from the start of radiography till the end of radiography, is detected. Further, the detected radiation dose for radiography is obtained through the network, and the plurality of radiation doses for radiography obtained from the radiography apparatuses, respectively, are stored and managed for each patient. Therefore, a radiation dose in pre-radiation, a radiation dose in failed radiography and the like, in which radiation is performed but a radiographic image for diagnosis is not obtained, is detected as a radiation dose for a patient, in addition to the radiation dose stored in such a manner to be related to a radiographic image. Therefore, it is possible to manage the radiation for the patient based on an accurate radiation dose.

When the radiation dose management means 20 includes the data analysis means 22 for judging whether the collected radiation amount, which is obtained by adding a plurality of radiation doses for radiography, is greater than or equal to a predetermined set threshold value, and when the radiography apparatus 10 includes the alert output means 15 for outputting an alert in the case that the data analysis means 22 has judged that the collected radiation dose is greater than or equal to the set threshold value, it is possible to output an alert in the case that the radiation dose for a patient increases or becomes close to or exceeds a predetermined level. Accordingly, it is possible to make a radiographer or a radiologist notice and pay attention to the radiation dose by outputting an alert.

When the radiation dose management means 20 has a function for managing the collected radiation dose for each region of the patient irradiated with radiation, it is possible to manage the radiation dose for each region of the human body.

Further, when the radiation dose detection means 14 detects the radiation dose for radiography by adding the radiation dose in pre-radiation, and/or failed radiography and the radiation dose in main radiography, it is possible to detect and manage, as the radiation dose for the patient, the radiation dose also in pre-radiation, failed radiography and the like, in which radiation is output but a radiographic image for diagnosis is not obtained, in addition to the radiation dose that is stored in such a manner to be related to the radiographic image.

The embodiment of the present invention is not limited to the above embodiments. For example, Figure 2 illustrates a radiography apparatus of decubitus type. However, the present invention may be applied to other kinds of radiography apparatuses, such as a chest X-ray apparatus, a mammography apparatus, a CT (computed tomography) apparatus, and an X-ray apparatus for dentistry.

## Claims

1. A radiography management system (1) for managing a radiation dose in radiography performed at each of a plurality of radiography apparatuses (10) that are connected to a network (2) in such a manner that data is transmittable through the network (2), the system (1) comprising:
a radiation dose detection means (14) that detects the dose of radiation in radiography that has irradiated a patient (S) from the start to the end of radiography to obtain a radiographic image at each of the plurality of radiography apparatuses (10); and
a radiation dose management means (20) that obtains, through the network (2), the dose of radiation detected by the radiation dose detection means (14), and that stores and manages, for each patient (S), a plurality of doses of radiation obtained from the plurality of radiography apparatuses (10) respectively in each time of radiography.

2. A radiography management system (1), as defined in Claim 1, wherein the radiation dose management means (20) includes a data analysis means (22) that judges whether a collected radiation dose obtained by adding the plurality of doses of radiation for each patient (S) is greater than or equal to a predetermined set threshold value, and wherein each of the plurality of radiography apparatuses (10) has an alert output means (15) that outputs an alert in the case that the data analysis means (22) has judged that the collected radiation dose is greater than or equal to the predetermined set threshold value.

3. A radiography management system (1), as defined in Claim 1 or 2, wherein the radiation dose management means (20) manages the collected radiation dose for each radiation-irradiated region of each patient (S).

4. A radiography management system (1), as defined in any one of Claims 1 to 3, wherein the radiation dose detection means (14) detects the dose of radiation in radiography by adding the dose of radiation in pre-radiation if pre-radiation has been performed, the dose of radiation in failed radiography if radiography has failed, the dose of radiation in main radiography in which the radiographic image is obtained.

5. A radiography management method for managing a radiation dose in radiography performed at each of a plurality of radiography apparatuses (10) that are connected to a network (2) in such a manner that data is transmittable through the network (2), the method comprising the steps of:
detecting the dose of radiation in radiography that has irradiated a patient (S) from the start to the end of radiography to obtain a radiographic image at each of the plurality of radiography apparatuses (10);
obtaining, through the network (2), the detected dose of radiation in radiography; and
storing and managing, for each patient (S), a plurality of doses of radiation obtained from the plurality of radiography apparatuses (10) respectively in each time of radiography.
